# EUROPÄISCHE PATENTSCHRIFT

(11) **EP 2 867 338 B1**
(45) Veröffentlichungstag und Bekanntmachung des Hinweises auf die Patenterteilung: **21.10.2015**
(21) Anmeldenummer: 13747793.1
(22) Anmeldetag: 01.08.2013
(51) Int. Cl.: C10G 9/36

(54) **VERFAHREN ZUM UMSETZEN VON KOHLENWASSERSTOFFEINSÄTZEN ZU OLEFINHALTIGEN PRODUKTSTRÖMEN DURCH THERMISCHES DAMPFSPALTEN**
METHOD FOR CONVERTING HYDROCARBON FEEDSTOCKS INTO OLEFINIC PRODUCT FLOWS BY MEANS OF THERMAL STEAM CRACKING
PROCÉDÉ DE CONVERSION DE CHARGES HYDROCARBONÉES EN DES COURANTS DE PRODUITS OLÉFINIQUES PAR CRAQUAGE THERMIQUE À LA VAPEUR D'EAU

(30) Priorität: 09.08.2012 EP 12005781
(43) Veröffentlichungstag der Anmeldung: 06.05.2015
(73) Patentinhaber: Linde AG, 80331 München (DE)
(72) Erfinder: SCHMIDT, Gunther, 82041 Deisenhofen (DE); FRITZ, Helmut, 81375 München (DE); WALTER, Stefanie, 82418 Seehausen (DE)
(86) Internationale Anmeldenummer: PCT/EP2013/002298
(87) Internationale Veröffentlichungsnummer: WO 2014/023407

(56) Entgegenhaltungen:
- US-A- 2 017 874
- US-A1- 2004 209 964
- US-A1- 2008 194 900
- US-A1- 2008 223 754
- US-B2- 6 743 961

## Beschreibung

Die vorliegende Erfindung betrifft ein Verfahren zur Umsetzung von Einsätzen aus Kohlenwasserstoffen durch thermisches Dampfspalten zu mindestens einem olefinhaltigen Produktstrom, welcher zumindest Ethylen und Propylen enthält, wobei die Einsätze in mindestens einem ersten Spaltofen und in mindesten einem zweiten Spaltofen wenigstens teilweise umgesetzt werden.

Beim thermischen Dampfspalten (auch als Dampfcracken oder Steamcracken bezeichnet, engl. Steam Cracking) handelt es sich um ein seit langem etabliertes Verfahren der Petrochemie. Die klassische Zielverbindung beim thermischen Dampfspalten ist das Ethylen (auch: Ethen), das eine wichtige Ausgangsverbindung für eine Reihe chemischer Synthesen darstellt.

Als Einsatz für das thermische Dampfspalten können sowohl Gase wie Ethan, Propan oder Butan und entsprechende Gemische als auch flüssige Kohlenwasserstoffe, wie beispielsweise Naphtha, und Kohlenwasserstoffgemische verwendet werden.

Zu den beim thermischen Dampfspalten im Einzelnen verwendeten Vorrichtungen und Reaktionsbedingungen und zu den ablaufenden Reaktionen sowie zu Einzelheiten der Raffinerietechnik sei auf entsprechende Artikel in Nachschlagewerken wie Zimmermann, H. und Walzl, R.: Ethylene. In: Ullmann's Encyclopedia of Industrial Chemistry. 6. Aufl. Weinheim: Wiley-VCH, 2005, und Irion, W.W. und Neuwirth, O.S.: Oil Refining. In: Ullmann's Encyclopedia of Industrial Chemistry. 6. Aufl. Weinheim: Wiley-VCH 2005, verwiesen. Verfahren zur Herstellung von Olefinen sind beispielsweise auch in der US 3 714 282 A und der US 6 743 961 B1 offenbart, wobeidie US 6 743 961 B2 ein Verfahren zur Herstellung von Olefinen beinhaltet, bei welchem Schweröl in einem milden Spaltprozess vorbehandelt wird bevor es in einen Ofen zum thermische Spalten geführt wird.

Desweitern sei hier die US 2004/209964 erwähnt, welche offenbart, dass Kohlenwasserstoffe mit einer Kohlenstoffzahl zwischen 15 und 30, die über eine Frischer-Tropsch-Synthese hergestellt und fraktioniert wurden, nach einer Hydrierung mild thermisch gespalten werden. Die US 2017874 A hingegen beinhaltet ein Verfahren, bei welchem aus Öl und Kraftstoffen über Olefine längerkettige Kohlenwasserstoffe hergestellt werden.

Zum thermischen Dampfspalten werden Spaltöfen eingesetzt. Die Spaltöfen sind, zusammen mit Quechenheit und nachgeschalteten Einrichtungen zur Aufarbeitung der gebildeten Produktgemische, in entsprechenden größeren Anlagen zur Olefinherstellung integriert, die im Rahmen dieser Anmeldung als "Steamcracker" bezeichnet werden.

Eine wichtige Kenngröße beim thermischen Dampfspalten ist die sogenannte Spaltschärfe (engl. Cracking Severity), welche die Spaltbedingungen bestimmt. Die Spaltbedingungen werden insbesondere beeinflusst von der Temperatur und der Verweilzeit sowie der Partialdrücke der Kohlenwasserstoffe und des Wasserdampfs. Auch die Zusammensetzung der als Einsatz verwendeten Kohlenwasserstoffgemische und die Bauart der verwendeten Spaltöfen beeinflussen die Spaltbedingungen. Aufgrund der wechselseitigen Einflüsse dieser Faktoren wird die Spaltbedingung normalerweise über das Verhältnis von Propylen (auch als Propen bezeichnet) zu Ethylen im Spaltgas festgelegt.

Beim thermischen Dampfspalten entstehen je nach Einsatzgemisch und Spaltbedingungen neben der klassischen Zielverbindung Ethylen mitunter beträchtliche Mengen an Nebenprodukten, die aus einem entsprechenden Produktstrom abgetrennt werden können. Hierbei handelt es sich unter anderem um niedere Alkene wie z.B. Propylen, und Butene sowie Diene, wie beispielsweise Butadiene, und sowie Aromaten wie z.B. Benzol, Toluol und Xylole. Diese besitzen einen vergleichsweise hohen wirtschaftlichen Wert, so dass ihre Bildung als sogenannte Mehrwertprodukte (engl. High Value Products) erwünscht ist.

Die US 6 743 961 B2 offenbart ein Verfahren zur Erzeugung von Olefinen, bei dem Rohöl in einer kombinierten Verdampfungs- und Spalteinheit teilweise verdampft wird. Der gebildete Dampf und die verbleibende Flüssigkeit werden bei unterschiedlichen Spaltbedingungen gespalten.

In der US 2004/209964 A1 wird ein Verfahren vorgeschlagen, bei dem ein Fischer-Tropsch-Produktstrom fraktioniert wird. Kohlenwasserstoffe unterschiedlicher Kettenlängen werden bei unterschiedlichen Spaltbedingungen gespalten.

Die vorliegende Erfindung stellt sich die Aufgabe, die Möglichkeiten zur Gewinnung von olefinhaltigen Produktgemischen aus Kohlenwasserstoffen durch thermisches Dampfspalten zu verbessern.

### Offenbarung der Erfindung

Die Erfindung schlägt vor diesem Hintergrund ein Verfahren zur Umsetzung von Einsätzen aus Kohlenwasserstoffen durch thermisches Dampfspalten zu mindestens einem olefinhaltigen Produktstrom, welcher zumindest Ethylen und Propylen enthält, wobei die Einsätze in mindestens einem ersten Spaltofen und in mindesten einem zweiten Spaltofen wenigstens teilweise umgesetzt werden, mit den Merkmalen der unabhängigen Patentansprüche vor. Bevorzugte Ausgestaltungen sind Gegenstand der Unteransprüche und der nachfolgenden Beschreibung.

### Vorteile der Erfindung

Erfindungsgemäß wird ein Verfahren vorgeschlagen, bei welchem ein Frischeinsatz in mindestens eine erste und eine zweite Frischeinsatz-Fraktion unterschiedlicher Zusammensetzung fraktioniert wird und die erste Frischeinsatz-Fraktion zumindest teilweise, vorzugsweise vollständig, in den ersten Spaltofen und die zweite Frischeinsatz-Fraktion zumindest teilweise, vorzugsweise vollständig in den zweiten Spaltofen geführt wird.

Unter Spaltofen wird im Rahmen dieser Erfindung eine Spalteinheit verstanden, in der die Spaltbedingungen festgelegt sind. Es ist möglich, dass an einem Gesamtofen eine Unterteilung in zwei oder mehr Spaltöfen vorliegt. Man spricht dann häufig von Ofenzellen. Mehrere, zu einem Gesamtofen gehörende Ofenzellen weisen in der Regel voneinander unabhängige Strahlungszonen und eine gemeinsame Konvektionszone sowie einen gemeinsamen Rauchabzug auf. In diesen Fällen kann jede Ofenzelle mit eigenen Spaltbedingungen betrieben werden. Jede Ofenzelle ist somit eine Spalteinheit und wird infolgedessen hier als Spaltofen bezeichnet. Der Gesamtofen weist dann mehrere Spalteinheiten oder, anders ausgedrückt, erweist mehrere Spaltöfen auf. Liegt nur eine Ofenzelle vor, ist diese die Spalteinheit und somit der Spaltofen. Spaltöfen können zu Gruppen zusammengefasst werden, welche beispielsweise mit dem gleichen Einsatz versorgt werden. Die Spaltbedingungen innerhalb einer Ofengruppe werden in der Regel gleich oder ähnlich eingestellt werden.

Gemäß der Erfindung weisen erste und zweite Frischeinsatz-Fraktion eine unterschiedliche Zusammensetzung auf. Damit wird betont, dass es sich bei der Teilung des Frischeinsatzes um eine Fraktionierung handelt und nicht um eine einfache Aufteilung in zwei Mengen. Bei einer Fraktionierung wird nach unterschiedlichen Komponenten getrennt. Nach der Fraktionierung finden sich also einige Komponenten des Frischeinsatzes überwiegend in der ersten Frischeinsatz-Fraktion und andere Komponenten des Frischeinsatzes finden sich überwiegend in der zweiten Frischeinsatz-Fraktion.

Bei der thermischen Spaltung von Kohlenwasserstoffen üblicher Zusammensetzung, wie beispielsweise Naphtha, unter milden Spaltbedingungen entsteht eine sehr große Menge an Pyrolysebenzin, welches aufgrund der großen Menge sehr schwierig in der Handhabung wird. Dies ist ein Resultat der vergleichsweise geringeren Umsetzung des Einsatzes im Spaltofen bei milden Spaltbedingungen. Milde Spaltbedingungen sind jedoch wünschenswert, da bei einer Spaltung unter milden Bedingungen im Verhältnis zum Frischeinsatz mehr Propylen entsteht als bei einer Spaltung unter normalen Spaltbedingungen, wie sie üblicherweise verwendet werden. Durch die erfindungsgemäße Fraktionierung des Frischeinsatzes und gezielte Führung in zugeordnete Spaltöfen wird der Frischeinsatz derartig aufgeteilt, dass in mindestens einem Spaltofen eine Spaltung unter milden Spaltbedingungen und in mindestens einem Spaltofen eine Spaltung unter üblichen Spaltbedingungen durchgeführt werden kann ohne dass dabei überaus große Mengen an Pyrolysebenzin entstehen. Die Mengen an Pyrolysebenzin bleiben vielmehr beherrschbar. Mit dem erfindungsgemäßen Verfahren wird es somit möglich, eine Anlage zum Steamcracken derartig zu betreiben, dass im Verhältnis zum Frischeinsatz mehr Propylen entsteht als in einer herkömmlichen Anlage, in welcher das erfindungsgemäße Verfahren nicht eingesetzt wird. Im Rahmen der Erfindung wurde diese Problematik und die vorgeschlagene Lösung erkannt.

Durch die erfindungsgemäße Fraktionierung des Frischeinsatzes und gezielte Führung in zugeordnete Spaltöfen wird es möglich, den Frischeinsatz gezielt aufzuteilen, so dass Spaltofeneinsatz und Spaltbedingungen optimal aufeinander abgestimmt werden können. Auf den ersten Blick erscheint es unsinnig eine Fraktionierung des Frischeinsatzes vorzunehmen, da diese mit einem erheblichen apparativen Aufwand und folglich mit hohen Investitionskosten verbunden ist. Es hat sich jedoch überraschenderweise gezeigt, dass die Vorteile, die sich aufgrund der nun möglichen Abstimmung von Spaltofeneinsatz und Spaltbedingungen einstellen, bei weitem die Nachteile überwiegen.

Die Vorgehensweisen, die notwendig sind, um den Frischeinsatz zu fraktionieren sind dem Fachmann bekannt. Es handelt sich dabei um in Steamcrackern übliche Maßnahmen zum Abtrennen und Aufarbeiten von Produkt- und Fraktionsströmen, die an die speziellen Eigenschaften des Frischeinsatzes angepasst werden.

Erfindungsgemäß enthält die zweite Frischeinsatz-Fraktion überwiegend Kohlenwasserstoffe, die maximal eine Kohlenstoffzahl von 5 aufweisen. Insbesondere enthält die zweite Frischeinsatz-Fraktion überwiegend Kohlenwasserstoffe, die eine Kohlenstoffzahl von 5 und/oder 4 aufweisen.

Weiterhin enthält die erste Frischeinsatz-Fraktion erfindungsgemäß überwiegend Kohlenwasserstoffe, die mindestens eine Kohlenstoffzahl von 6 aufweisen.

Wird der Frischeinsatz derartig aufgeteilt, kann eine Anlage zum Steamcracken ganz besonders vorteilhaft betrieben werden und es entsteht im Verhältnis zum Frischeinsatz deutlich mehr Propylen als in einer herkömmlichen Anlage.

Der Begriff "überwiegen" wird im Rahmen dieser Anmeldung verwendet, um deutlich zu machen, dass der Einsatz oder die Fraktion nicht ausschließlich aus Kohlenwasserstoffen mit der angegeben Kohlenstoffzahl besteht, sondern neben den Kohlenwasserstoffen der angegeben Kohlenstoffzahl auch Kohlenwasserstoffe mit anderen Kohlenstoffzahlen sowie andere Verunreinigungen vorhanden sein können. Bei der Trennung und Aufarbeitung des Frischeinsatzes, des Produktstroms und/oder den Fraktionen bleiben stets Reste der Komponente(n) in dem Produktstrom beziehungsweise in der Fraktion. Auch andere Verunreinigungen bleiben bestehen, so dass ein bearbeiteter Produkt- oder Fraktionsstrom stets Rückstände enthält. Da der Aufwand für Abtrennung und Aufarbeitung mit der zu erzielenden Reinheit extrem stark ansteigt, hängt es von wirtschaftlichen Faktoren ab, welchen Anteil an Rückständen in einem Strom enthalten sein dürfen. Wie hoch dieser Anteil ist, muss nach wirtschaftlichen Gesichtspunkten abgewogen werden. Als groben Richtwert für den Anteil an unerwünschten Kohlenwasserstoffen und anderen Verunreinigungen wird in der Regel gelten, dass diese mit maximal 30 bis 40 Gewichtsprozent in dem Produktstrom und/oder in der Fraktion enthalten sein dürfen. Meist wird sogar ein Maximalwert von 15 Gewichtsprozent oder weniger erreicht. Für die Frischeinsatz-Fraktionen gilt somit, dass diese die gewünschten Kohlenwasserstoffe mit mindestens 60 Gewichtsprozent, bevorzugt mindestens 80 Gewichtsprozent und weiter bevorzugt mindestens 90 Gewichtsprozent und besonders bevorzugt mindestens 95 Gewichtsprozent und ganz besonders bevorzugt mindestens 98 Gewichtsprozent enthalten. Die eben gesagten Grenzen gelten auch für die rückgeführten Fraktionen (siehe unten).

Erfindungsgemäß herrschen im zweiten Spaltofen Spaltbedingungen, die zu einem Verhältnis von Propylen zu Ethylen von 0,7 bis 1,6 kg/kg, bevorzugt von 0,8 bis 1,4 kg/kg, besonders bevorzugt von 0,85 bis 1,2 kg/kg am Spaltofenaustritt führen. Wird der Einsatz bei milden Spaltbedingungen umgesetzt, zeigen sich die vorgenannten Vorteile der Erfindung besonders ausgeprägt. Von Vorteil sind auch Spaltbedingungen, die zu einem Verhältnis von Propylen zu Ethylen am Spaltofenaustritt führen von 0,75 bis 1,5 kg/kg oder von 0,8 bis 1,2 kg/kg oder von 0,85 bis 1,15 kg/kg oder welche sogar in dem engen Bereich von 0,9 bis 1,1 kg/kg liegen.

Weiterhin ist es erfindungsgemäß vorgesehen, dass im ersten Spaltofen Spaltbedingungen herrschen, die zu einem Verhältnis von Propylen zu Ethylen von 0,25 bis 0,85 kg/kg, bevorzugt von 0,3 bis 0,75 kg/kg, besonders bevorzugt von 0,4 bis 0,65 kg/kg am Spaltofenaustritt führen, wobei die erreichten Werte für das Verhältnis von Propylen zu Ethylen des zweiten Spaltofens über denen des ersten Spaltofens liegen.

Durch das Betreiben von mindestens zwei Spaltöfen bei den eben genannten verschiedenen Spaltbedingungen stellen sich ganz besondere Vorteile ein, da die Spaltbedingungen in beiden Spaltöfen an den jeweiligen Einsatz angepasst werden kann. So zeichnen sich die Spaltbedingungen im zweiten Spaltofen dadurch aus, dass mit diesem die angegebenen sehr hohen Werte für das Verhältnis von Propylen zu Ethylen erreicht werden. Im ersten Spaltofen wird hingegen der Einsatz bei üblichen Spaltbedingungen umgesetzt. Durch die Anpassung der Spaltbedingungen an den jeweiligen Einsatz wird erreicht, dass die Pyrolysebenzin-Fraktion mengenmäßig beherrschbar bleibt. Auch bilden sich im zweiten Spaltofen bei milden Bedingungen geringere Mengen an Pyrolyseöl als im ersten Spaltofen, jedoch sind auch die im ersten Spaltofen die entstehenden Mengen an Pyrolyseöl beherrschbar.

Dabei liegen die erreichten Werte für das Verhältnis von Propylen zu Ethylen des zweiten Spaltofens mit Vorteil um mindestens 0,1 kg/kg, vorzugsweise um mindestens 0,15 kg/kg, besonders bevorzugt um mindestens 0,2 kg/kg über denen des ersten Spaltofens.

In einer besonders vorteilhaften Ausgestaltung der Erfindung werden in den zweiten Spaltofen eine oder mehrere Fraktionen, welche aus dem Produktstrom gewonnen werden und welche Kohlenwasserstoffe enthalten, die maximal eine Kohlenstoffzahl von 5 aufweisen, rückgeführt. Dies hat den Vorteil, dass sich die Menge an Einsatz, die in den mindestens einen zweiten Spaltofen geführt wird, erhöht. Dabei ist die rückgeführte Fraktion derartig gewählt, dass sie die Vorteile des erfindungsgemäßen Verfahrens aufgrund ihrer Zusammensetzung unterstützt. Besonders ausgeprägt ist diese Unterstützung für Fraktionen mit Kohlenwasserstoffen, die eine Kohlenstoffzahl von 5 und/oder 4 aufweisen.

Weiterhin ist es von Vorteil wenn in den ersten Spaltofen eine oder mehrere Fraktionen, welche aus dem Produktstrom gewonnen werden und welche Kohlenwasserstoffe enthalten, die mindestens eine Kohlenstoffzahl von 6 aufweisen, rückgeführt werden. Derartige Fraktionen können aufgrund ihrer Zusammensetzung im ersten Spaltofen gut zu Wertprodukten aufgespalten werden.

In einer weiteren vorteilhaften Ausgestaltung der Erfindung wird dem zweiten Spaltofen neben der zweiten Frischeinsatz-Fraktion ein weiterer Frischeinsatz zugeführt, welcher überwiegend aus Kohlenwasserstoffen besteht, die maximal eine Kohlenstoffzahl von 5 aufweisen. Ein solcher weitere Frischeinsatz kann beispielsweise in einer Raffinerie oder bei der Erdgasgewinnung anfallen. Aufgrund seiner Beschaffenheit ist er als Einsatz im zweiten Spaltofen bei milden Spaltbedingungen sehr gut geeignet. Weiterhin kann es ebenso von Vorteil sein, dem ersten Spaltofen neben der ersten Frischeinsatz-Fraktion einen weiteren Frischeinsatz zuzugeben.

Wie eingangs erläutert, ergibt sich das Verhältnis von Propylen zu Ethylen beim thermischen Dampfspalten aus einer Reihe unterschiedlicher Einflussfaktoren, bei denen die Spaltofenaustrittstemperatur, d.h. die Temperatur eines Produktstroms beim Verlassen der verwendeten Reaktorschlange (engl. Coil Output Temperature), eine wichtige Rolle spielt. Die Spaltofenaustrittstemperatur für die Umsetzung im der zweiten Spaltofen liegt vorteilhafterweise zwischen 680 °C und 820 °C, bevorzugt zwischen 700 °C und 800 °C und weiter bevorzugt zwischen 710 °C und 780°C und besonders bevorzugt zwischen 720 °C und 760 °C während die die Spaltofenaustrittstemperatur für die Umsetzung im ersten Spaltofen zwischen vorteilhafterweise zwischen 800 °C und 1000 °C, bevorzugt zwischen 820 °C und 950 °C und besonders bevorzugt zwischen 840 °C und 900 °C liegt. Dabei ist die Spaltofenaustrittstemperatur am ersten Spaltofen stets höher als am zweiten Spaltofen.

Dabei liegt die Spaltofenaustrittstemperatur für die Umsetzung im ersten Spaltofen bevorzugt um mindestens 10 °C, besonders bevorzugt um mindestens 15°C und ganz besonders bevorzugt um mindestens 20 °C über der Spaltofenaustrittstemperatur für die Umsetzung im zweiten Spaltofen.

Im zweiten Spaltofen kann ferner eine geringere Dampfverdünnung als im ersten verwendet werden. Dies verringert die notwendige Verdünnungsdampfmenge und spart Energie ein. Eine geringere Dampfverdünnung im zweiten Spaltofen ist jedoch nicht notwendig, damit sich die wesentlichen Vorteil der Erfindung zeigen. Vorteilhafterweise werden im zweiten Spaltofen 0,15 bis 0,8 kg Wasserdampf pro kg Kohlenwasserstoff im Einsatz verwendet während im ersten Spaltofen 0,3 bis 1,5 kg Wasserdampf pro kg Kohlenwasserstoff im Einsatz verwendet werden.

Auch können mit Vorteil in dem Produktstrom enthaltene, insbesondere gesättigte Kohlenwasserstoffe mit einer Kohlenstoffzahl von 2 bis 3 mit Vorteil mittels thermischen Dampfspalten in einem Spaltofen für gasförmigen Einsatz umgesetzt werden. Dazu werden die gesättigten gasförmigen Kohlenwasserstoffe aus dem Produktstrom gewonnen und in den Spaltofen für gasförmigen Einsatz rückgeführt und dort umgesetzt.

Als Frischeinsatz für die Frischeinsatz-Fraktionierung können sowohl Gase oder Gasfraktionen wie Ethan, Propan oder Butan und entsprechende Gemische und Kondensate als auch flüssige Kohlenwasserstoffe und Kohlenwasserstoffgemische verwendet werden. Die genannten Gasgemische und Kondensate umfassen insbesondere sogenannte Erdgaskondensate (engl. Natural Gas Liquids, NGL). Die flüssigen Kohlenwasserstoffe und Kohlenwasserstoffgemische können beispielsweise aus der sogenannten Benzinfraktion von Rohöl stammen. Bei derartigen Rohbenzinen bzw. Naphthas (NT) und Kerosin handelt es sich um Gemische aus vorzugsweise gesättigten Verbindungen mit Siedepunkten zwischen 35 und 210 °C.

Als Frischeinsatz sind flüssige Kohlenwasserstoffe ganz besonders von Vorteil. Insbesondere werden als Frischeinsatz Erdgaskondensate und/oder Rohölfraktionen und/oder hiervon abgeleiteten Gemische verwendet.

Vorteilhafterweise umfasst die Erfindung damit die Verwendung von Kohlenwasserstoffgemischen mit einem Siedebereich von bis zu 600 °C als Frischeinsatz. Innerhalb dieses Gesamtbereichs können auch Kohlenwasserstoffgemische mit abweichenden Siedebereichen verwendet werden, beispielsweise mit Siedebereichen von bis zu 360 °C oder von bis zu 240 °C. Die Reaktionsbedingungen im Spaltofen werden hierbei auf die jeweils eingesetzten Kohlenwasserstoffgemische abgestimmt.

So kann die Erfindung mit Vorteil jedoch auch mit beliebigen anderen Frischeinsätzen verwendet werden, die vergleichbare Eigenschaften aufweisen, wie beispielsweise biogene oder/und synthetische Kohlenwasserstoffe.

### Kurze Beschreibung der Zeichnung

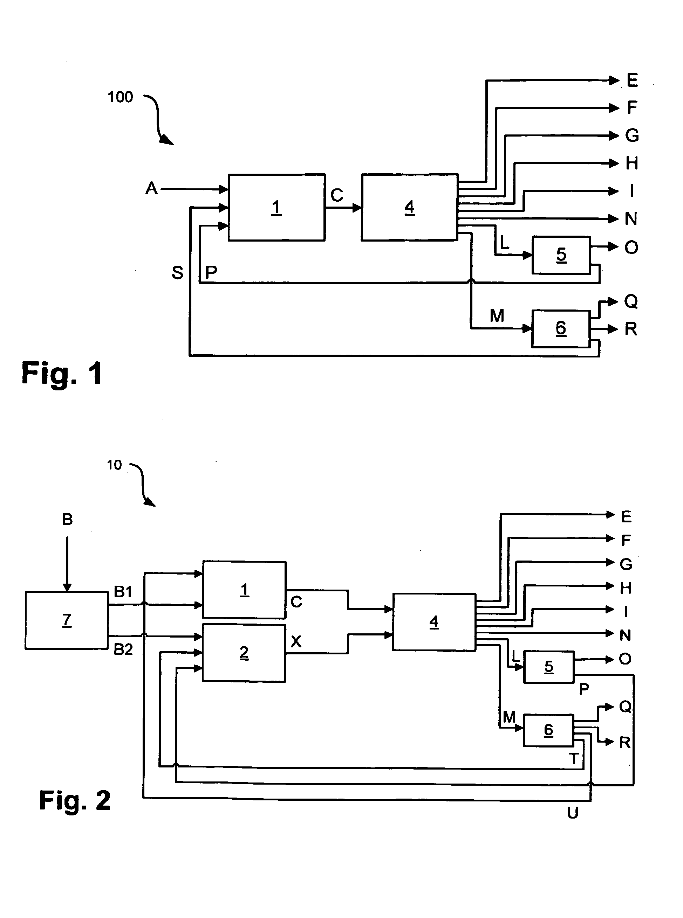

Das erfindungsgemäße Verfahren in besonders vorteilhafter Ausgestaltung soll anhand der Prozessschaubilder, welche die wesentlichen Prozessschritte schematisch zeigen, näher erklärt werden. Zum besseren Verständnis wird zuerst anhand von Figur 1 das bekannte Verfahren dargelegt.

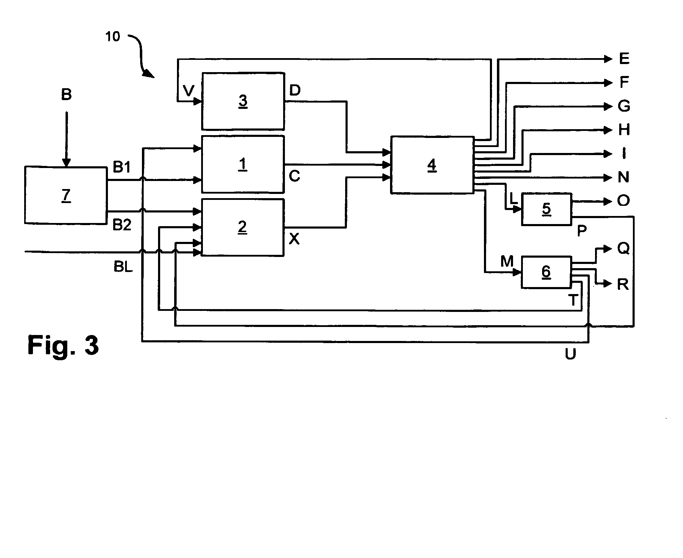

Figur 1 zeigt dazu in schematischer Darstellung ein bekanntes Vorgehen zur Olefinherstellung. Figur 2 zeigt in schematischer Darstellung die wesentlichen Schritte des erfindungsgemäßen Verfahrens in besonders vorteilhafter Ausgestaltung und Figur 3 zeigt, ebenfalls schematisch, die wesentlichen Schritte einer besonders vorteilhaften Ausgestaltung der Erfindung. In den Figuren tragen einander entsprechende Elemente identische Bezugzeichen.

Das schematische Prozessschaubild 100 der Figur 1 für das bekannte Verfahren beinhaltet einen Spaltofen 1, in welchen der Frischeinsatz A (beispielsweise Naphtha) sowie die rückgeführten Fraktionen S und P als Kohlenwasserstoffeinsatz geführt werden. Im Spaltofen 1 wird der Kohlenwasserstoffeinsatz in Konvektions- und Strahlungszone erwärmt und umgesetzt. In den Spaltofen wird Wasserdampf zugegeben, meist 0,5 bis 1 kg Prozessdampf pro kg Kohlenwasserstoff. Aus dem Spaltofen 1 tritt ein Produktstrom C aus, der direkt am Austritt aus dem Spaltofen auch als Spaltproduktstrom bezeichnet wird. Beim Austritt aus dem Spaltofen weist dieser Spaltproduktstrom eine Temperatur aus, die normalerweise zwischen 840 °C und 900°C liegt. Das Verhältnis von Propylen zu Ethylen liegt in der Regel bei 0,35 bis 0,6 kg/kg. Nach einem ersten Quenchen (nicht dargestellt) wird der Produktstrom wird in einer Aufarbeitungseinheit (englisch: processing unit) 4 verarbeitet. Aus der Aufarbeitungseinheit werden als wesentliche Produktfraktionen E bis N folgende Fraktionen gewonnen: Wasserstoff E, Ablauge F, Methan G, Ethylen H, Propylen I, gasförmige Kohlenwasserstoffe L mit einer Kohlenstoffzahl von 4, Pyrolysebenzin M und Pyrolyseöl N. Die gasförmigen Kohlenwasserstoffe L mit einer Kohlenstoffzahl von 4 werden in einer C4-Aufarbeitungseinheit 5, welche für die Verarbeitung von Kohlenwasserstoffen mit einer Kohlenstoffzahl von 4 benutzt wird, weiter behandelt. Eine solche C4-Aufarbeitungseinheit 5 behandelt die Fraktion mit einer Kohlenstoffzahl von 4 derartig weiter, dass Butadien O abgeführt werden können. Die übrigen Kohlenwasserstoff mit einer Kohlenstoffzahl von 4 stellen eine Fraktion P dar, die in den Spaltofen 1 rückgeführt wird. Das Pyrolysebenzin M, welches Kohlenwasserstoffe mit einer Kohlenstoffzahl von 5 und mehr umfasst, wird in einer Pyrolysebenzin-Aufarbeitungseinheit 6 weiterverarbeitet und es werden Aromate Q und Kohlenwasserstoffe R mit einer Kohlenstoffzahl von beispielsweise mehr als 9 abgeführt. Die übrigen Kohlenwasserstoffe mit einer Kohlenstoffzahl von 5 und mehr werden als Fraktion S in den Spaltofen 1 rückgeführt. Die Aufarbeitungseinheit 4 sowie die C4-Aufarbeitungseinheit 5 und die Pyrolysebenzin-Aufarbeitungseinheit 6 umfassen übliche Einheiten zur Weiterverarbeitung des Produktstroms beziehungsweise der Produktfraktionen, welche zur Ausführung verschiedener Prozessschritte dienen, wie beispielsweise Verdichtung, Kondensation und Abkühlung, Trocknung, Destillation und Fraktionierung, Extraktion und Hydrierung. Die Prozessschritte sind in Olefinanlagen üblich und dem Fachmann bekannt.

Das schematische Prozessschaubild 10 der Figur 2 zeigt nun das erfindungsgemäße Verfahren in einer besonders vorteilhaften Ausgestaltung und seine wesentlichen Prozessschritte. Zusätzlich zu dem Spaltofen 1 ist hier ein zweiter Spaltofen 2 vorhanden sowie eine Frischeinsatz-Fraktionierungseinheit 7. Ein Frischeinsatz B (beispielsweise Naphtha) wird nun in der Frischeinsatz-Fraktionierungseinheit 7 fraktioniert und die erste Frischeinsatz-Fraktion B1 wird in den ersten Spaltofen 1 geführt, während die zweite Frischeinsatz-Fraktion B2 in den zweiten Spaltofen 2 geführt wird. Für die Prozesse zur Fraktionierung des Frischeinsatzes werden die üblichen Methoden zur Separation und Behandlung von Kohlenwasserstoffströmen verwendet, wie sie aus Olefinanlagen aus Raffinerien bekannt sind. Diese kennt der Fachmann und er weiß sie einzusetzen. In den ersten Spaltofen 1 werden mit Vorteil zusätzlich eine Fraktion U und in den zweiten Spaltofen 2 zusätzlich die Fraktionen T und P rückgeführt (näheres siehe weiter unten). Aus dem ersten Spaltofen 1 tritt wiederum der Spaltproduktstrom C mit den oben genannten Eigenschaften aus. Aus dem zweiten Spaltofen 2 tritt der Spaltproduktstrom X aus. Der Spaltproduktstrom X weist eine Temperatur aus, die vorteilhafterweise zwischen 700 °C und 800°C liegt. Das Verhältnis von Propylen zu Ethylen liegt dabei vorteilhafterweise zwischen 0,7 bis 1,5 kg/kg. Die Produktströme C und X werden in der Aufarbeitungseinheit 4 weiterverarbeitet und an geeigneter Stelle zu einem gemeinsamen Produktstrom zusammengeführt. Die Prozesse zur Weiterbehandlung und Aufarbeitung in der Aufarbeitungseinheit 4 sind bekannt und wurden eben beschrieben. So führt die Aufarbeitungseinheit 4 auch, wie eben beschrieben, zu den Produktfraktionen E bis N. Auch die Produktfraktionen L und M werden, wie eben beschrieben, in den speziellen Aufarbeitungseinheiten 5 und 6 weiterbehandelt. Im Gegensatz zu dem in Figur 1 beschrieben Verfahren wird nun vorteilhafterweise auch die Fraktion P, welche Kohlenwasserstoffe mit einer Kohlenstoffzahl von 4 enthält, nicht in den Spaltofen 1 sondern in den zweiten Spaltofen 2 rückgeführt. In der Pyrolysebenzin-Aufarbeitungseinheit 6 werden neben den oben erwähnten Fraktionen Q und R die Fraktionen T und U gewonnen. Die Fraktion T, welche Kohlenwasserstoffe mit einer Kohlenstoffzahl von 5 enthält, wird vorteilhafterweise in den zweiten Spaltofen 2 rückgeführt, während die Fraktion U, welche Kohlenwasserstoffe mit einer Kohlenstoffzahl von 6 und mehr, insbesondere zwischen 6 und 9, enthält, vorteilhafterweise in den ersten Spaltofen 1 rückgeführt wird.

Eine besonders vorteilhafte Ausgestaltung der Erfindung beinhaltet Figur 3. Figur 3 weist das gleiche schematische Prozessschaubild auf, wie es auch Figur 2 zeigt. Ergänzt ist dieses um einen Spaltofen 3 für gasförmigen Einsatz, in welche eine Fraktion V als Einsatz geführt wird. Die Fraktion V enthält gesättigte gasförmige Kohlenwasserstoffe mit einer Kohlenstoffzahl von 2 oder 3, welche ebenfalls in der Aufarbeitungseinheit 4 gewonnen werden. Zusätzlich wird hier auch ein weiterer Frischeinsatz BL, welcher überwiegend aus Kohlenwasserstoffen mit einer Kohlenstoffzahl von maximal 5 besteht, dem zweiten Spaltofen 2 zugeführt.

### Bezugszeichenliste

- 1: Spaltofen (normale Spaltbedingungen)
- 2: Spaltofen (milde Spaltbedingungen)
- 3: Spaltofen für gasförmigen Einsatz
- 4: Aufarbeitungseinheit
- 5: C4-Aufarbeitungseinheit
- 6: Pyrolysebenzin-Aufarbeitungseinheit
- 7: Frischeinsatz-Fraktionierungseinheit

- 10: schematisches Prozessschaubild für ein bekanntes Verfahren

- 100: schematisches Prozessschaubild für das erfindungsgemäße Verfahren in einer besonders vorteilhaften Ausgestaltung

- A, B, BL: Frischeinsatz
- B1, B2: Frischeinsatz-Fraktionen
- C, D, X: Produktströme
- E-V: Produktfraktionen

## Patentansprüche

1. Verfahren zur Umsetzung von Einsätzen aus Kohlenwasserstoffen durch thermisches Dampfspalten zu mindestens einem olefinhaltigen Produktstrom, welcher zumindest Ethylen und Propylen enthält, wobei die Einsätze in mindestens einem ersten Spaltofen (1) und in mindesten einem zweiten Spaltofen (2) wenigstens teilweise umgesetzt werden, wobei ein Frischeinsatz (B) in mindestens eine erste und eine zweite Frischeinsatz-Fraktion (B1, B2) unterschiedlicher Zusammensetzung fraktioniert wird und die erste Frischeinsatz-Fraktion (B1) zumindest teilweise in den ersten Spaltofen (1) und die zweite Frischeinsatz-Fraktion (B2) zumindest teilweise in den zweiten Spaltofen (2) geführt wird, **dadurch gekennzeichnet, dass** die zweite Frischeinsatz-Fraktion (B2) überwiegend Kohlenwasserstoffe enthält, die maximal eine Kohlenstoffzahl von 5 aufweisen, dass die erste Frischeinsatz-Fraktion (B1) überwiegend Kohlenwasserstoffe enthält, die mindestens eine Kohlenstoffzahl von 6 aufweisen, dass im zweiten Spaltofen (2) Spaltbedingungen herrschen, die zu einem Verhältnis von Propylen zu Ethylen von 0,7 bis 1,6 kg/kg am Spaltofenaustritt führen, und dass im ersten Spaltofen (1) Spaltbedingungen herrschen, die zu einem Verhältnis von Propylen zu Ethylen von 0,25 bis 0,85 kg/kg am Spaltofenaustritt führen, wobei die erreichten Werte für das Verhältnis von Propylen zu Ethylen des zweiten Spaltofens (2) über denen des ersten Spaltofens (1) liegen.

2. Verfahren nach Anspruch 1, **dadurch gekennzeichnet, dass** im zweiten Spaltofen (2) Spaltbedingungen herrschen, die zu einem Verhältnis von Propylen zu Ethylen von 0,8 bis 1,4 kg/kg, bevorzugt von 0,85 bis 1,2 kg/kg am Spaltofenaustritt führen.

3. Verfahren nach einem der Ansprüche 1 oder 2, **dadurch gekennzeichnet, dass** im ersten Spaltofen (1) Spaltbedingungen herrschen, die zu einem Verhältnis von Propylen zu Ethylen von 0,3 bis 0,75 kg/kg, bevorzugt von 0,4 bis 0,65 kg/kg am Spaltofenaustritt führen, wobei die erreichten Werte für das Verhältnis von Propylen zu Ethylen des zweiten Spaltofens (2) über denen des ersten Spaltofens (1) liegen.

4. Verfahren nach einem der Ansprüche 1 bis 3, bei dem die erreichten Werte für das Verhältnis von Propylen zu Ethylen des zweiten Spaltofens (2) um mindestens 0,1 kg/kg, vorzugsweise um mindestens 0,15 kg/kg, besonders bevorzugt um mindestens 0,2 kg/kg über denen des ersten Spaltofens (1) liegen.

5. Verfahren nach einem der Ansprüche 1 bis 4, **dadurch gekennzeichnet, dass** in den zweiten Spaltofen (2) eine oder mehrere Fraktionen (P, T), welche aus dem Produktstrom gewonnen werden und welche Kohlenwasserstoffe enthalten, die maximal eine Kohlenstoffzahl von 5 aufweisen, rückgeführt werden.

6. Verfahren nach einem der Ansprüche 1 bis 5, **dadurch gekennzeichnet, dass** in den ersten Spaltofen (1) eine oder mehrere Fraktionen (U), welche aus dem Produktstrom gewonnen werden und welche Kohlenwasserstoffe enthalten, die mindestens eine Kohlenstoffzahl von 6 aufweisen, rückgeführt werden.

7. Verfahren nach einem der Ansprüche 1 bis 6, **dadurch gekennzeichnet, dass** dem zweiten Spaltofen (2) neben der zweiten Frischeinsatz-Fraktion (B2) ein weiterer Frischeinsatz (BL) zugeführt wird, welcher überwiegend aus Kohlenwasserstoffen besteht, die maximal eine Kohlenstoffzahl von 5 aufweisen.

8. Verfahren nach einem der Ansprüche 1 bis 7, bei welchem die Spaltofenaustrittstemperatur für die Umsetzung im zweiten Spaltofen (2) zwischen 680 °C und 820 °C, bevorzugt zwischen 700 °C und 800 °C und weiter bevorzugt zwischen 710 °C und 780°C und besonders bevorzugt zwischen 720 °C und 760 °C liegt und die Spaltofenaustrittstemperatur für die Umsetzung im ersten Spaltofen (1) zwischen 800 °C und 1000 °C, bevorzugt zwischen 820 °C und 950 °C und besonders bevorzugt zwischen 840 °C und 900 °C liegt, wobei die Spaltofenaustrittstemperatur des ersten Spaltofens (1) über der des zweiten Spaltofens (2) liegt.

9. Verfahren nach Anspruch 8, bei welchem die Spaltofenaustrittstemperatur für die Umsetzung im ersten Spaltofen (1) um mindestens 10 °C, bevorzugt um mindestens 15°C, besonders bevorzugt um mindestens 20 °C über der Spaltofenaustrittstemperatur für die Umsetzung im zweiten Spaltofen (2) liegt.

10. Verfahren nach einem der Ansprüche 1 bis 9, bei welchem im ersten Spaltofen (1) 0,3 bis 1,5 kg Wasserdampf pro kg Kohlenwasserstoffeinsatz und im zweiten Spaltofen (2) 0,15 bis 0,8 kg Wasserdampf pro kg Kohlenwasserstoffeinsatz verwendet wird.

11. Verfahren nach einem der Ansprüche 1 bis 10, bei welchem aus dem Produktstrom zumindest eine Fraktion (V), welche überwiegend Kohlenwasserstoffe mit einer Kohlenstoffzahl von 2 oder 3 aufweist, gewonnen wird und in einem Spaltofen (3) für gasförmigen Einsatz zumindest teilweise umgesetzt wird.

12. Verfahren nach einem der Ansprüche 1 bis 11, **dadurch gekennzeichnet, dass** als Frischeinsatz (B) Erdgaskondensate und/oder Rohölfraktionen, insbesondere Naphtha, und/oder synthetische und/oder biogene Kohlenwasserstoffe und/oder hiervon abgeleiteten Gemische verwendet werden.

## Claims

1. Process for converting inputs composed of hydrocarbons by thermal steamcracking to at least one olefin-containing product stream comprising at least ethylene and propylene, with at least partial conversion of the inputs in at least one first cracking furnace (1) and in at least one second cracking furnace (2), wherein a fresh input (B) is fractionated into at least one first and one second fresh input fraction (B1, B2) of different composition and the first fresh input fraction (B1) is conducted at least partly into the first cracking furnace (1) and the second fresh input fraction (B2) at least partly into the second cracking furnace (2), **characterized in that** the second fresh input fraction (B2) comprises predominantly hydrocarbons having a maximum carbon number of 5, **in that** the first fresh input fraction (B1) comprises predominantly hydrocarbons having a carbon number of at least 6, **in that** cracking conditions that lead to a ratio of propylene to ethylene of 0.7 to 1.6 kg/kg at the cracking furnace exit exist in the second cracking furnace (2), and **in that** cracking conditions that lead to a ratio of propylene to ethylene of 0.25 to 0.85 kg/kg at the cracking furnace exit exist in the first cracking furnace (1), the values attained for the ratio of propylene to ethylene in the second cracking furnace (2) being above those in the first cracking furnace (1).

2. Process according to Claim 1, **characterized in that** cracking conditions that lead to a ratio of propylene to ethylene of 0.8 to 1.4 kg/kg and preferably of 0.85 to 1.2 kg/kg at the cracking furnace exit exist in the second cracking furnace (2).

3. Process according to either of Claims 1 and 2, **characterized in that** cracking conditions that lead to a ratio of propylene to ethylene of 0.3 to 0.75 kg/kg and preferably of 0.4 to 0.65 kg/kg at the cracking furnace exit exist in the first cracking furnace (1), the values attained for the ratio of propylene to ethylene in the second cracking furnace (2) being above those in the first cracking furnace (1).

4. Process according to any of Claims 1 to 3, in which the values attained for the ratio of propylene to ethylene in the second cracking furnace (2) are at least 0.1 kg/kg above, preferably at least 0.15 kg/kg above and more preferably at least 0.2 kg/kg above those in the first cracking furnace (1).

5. Process according to any of Claims 1 to 4, **characterized in that** one or more fractions (P, T) which are obtained from the product stream and which comprise hydrocarbons having a maximum carbon number of 5 are recycled into the second cracking furnace (2).

6. Process according to any of Claims 1 to 5, **characterized in that** one or more fractions (U) which are obtained from the product stream and which comprise hydrocarbons having a carbon number of at least 6 are recycled into the first cracking furnace (1).

7. Process according to any of Claims 1 to 6, **characterized in that** the second cracking furnace (2) is supplied not only with the second fresh input fraction (B2) but also with a further fresh input (BL) consisting predominantly of hydrocarbons having a maximum carbon number of 5.

8. Process according to any of Claims 1 to 7, in which the cracking furnace exit temperature for the conversion in the second cracking furnace (2) is between 680°C and 820°C, preferably between 700°C and 800°C and further preferably between 710°C and 780°C and more preferably between 720°C and 760°C, and the cracking furnace exit temperature for the conversion in the first cracking furnace (1) is between 800°C and 1000°C, preferably between 820°C and 950°C and more preferably between 840°C and 900°C, the cracking furnace exit temperature of the first cracking furnace (1) being above that of the second cracking furnace (2).

9. Process according to Claim 8, in which the cracking furnace exit temperature for the conversion in the first cracking furnace (1) is at least 10°C above, preferably at least 15°C above, more preferably at least 20°C above, the cracking furnace exit temperature for the conversion in the second cracking furnace (2).

10. Process according to any of Claims 1 to 9, in which 0.3 to 1.5 kg of steam per kg of hydrocarbon input is used in the first cracking furnace (1), and 0.15 to 0.8 kg of steam per kg of hydrocarbon input in the second cracking furnace (2).

11. Process according to any of Claims 1 to 10, in which at least one fraction (V) comprising predominantly hydrocarbons having a carbon number of 2 or 3 is obtained from the product stream and at least partly converted in a cracking furnace (3) for gaseous input.

12. Process according to any of Claims 1 to 11, **characterized in that** the fresh input (B) used comprises natural gas condensates and/or crude oil fractions, especially naphtha, and/or synthetic and/or biogenic hydrocarbons and/or mixtures derived therefrom.

## Revendications

1. Procédé de transformation d'alimentations d'hydrocarbures par clivage thermique à la vapeur en au moins un courant de produits contenant des oléfines, qui contient au moins de l'éthylène et du propylène, les alimentations étant au moins partiellement transformées dans au moins un premier four de clivage (1) et dans au moins un second four de clivage (2), une alimentation fraîche (B) étant fractionnée en au moins une première et une seconde fraction d'alimentation fraîche (B1, B2) de composition différente, et la première fraction d'alimentation fraîche (B1) étant introduite au moins partiellement dans le premier four de clivage (1) et la seconde fraction d'alimentation fraîche (B2) étant introduite au moins partiellement dans le second four de clivage (2), **caractérisé en ce que** la seconde fraction d'alimentation fraîche (B2) contient principalement des hydrocarbures qui présentent au plus un nombre de carbone de 5, **en ce que** la première fraction d'alimentation fraîche (B1) contient principalement des hydrocarbures qui présentent au moins un nombre de carbone de 6, **en ce que** des conditions de clivage qui conduisent à un rapport entre le propylène et l'éthylène de 0,7 à 1,6 kg/kg à la sortie du four de clivage règnent dans le second four de clivage (2), et **en ce que** des conditions de clivage qui conduisent à un rapport entre le propylène et l'éthylène de 0,25 à 0,85 kg/kg à la sortie du four de clivage règnent dans le premier four de clivage (1), les valeurs atteintes pour le rapport entre le propylène et l'éthylène du second four de clivage (2) étant supérieures à celles du premier four de clivage (1).

2. Procédé selon la revendication 1, **caractérisé en ce que** des conditions de clivage qui conduisent à un rapport entre le propylène et l'éthylène de 0,8 à 1,4 kg/kg, de préférence de 0,85 à 1,2 kg/kg, à la sortie du four de clivage règnent dans le second four de clivage (2).

3. Procédé selon l'une quelconque des revendications 1 ou 2, **caractérisé en ce que** des conditions de clivage qui conduisent à un rapport entre le propylène et l'éthylène de 0,3 à 0,75 kg/kg, de préférence de 0,4 à 0,65 kg/kg, à la sortie du four de clivage règnent dans le premier four de clivage (1), les valeurs atteintes pour le rapport entre le propylène et l'éthylène du second four de clivage (2) étant supérieures à celles du premier four de clivage (1).

4. Procédé selon l'une quelconque des revendications 1 à 3, **caractérisé en ce que** les valeurs atteintes pour le rapport entre le propylène et l'éthylène du second four de clivage (2) sont supérieures d'au moins 0,1 kg/kg, de préférence d'au moins 0,15 kg/kg, de manière particulièrement préférée d'au moins 0,2 kg/kg, à celles du premier four de clivage (1).

5. Procédé selon l'une quelconque des revendications 1 à 4, **caractérisé en ce qu'**une ou plusieurs fractions (P, T) qui sont obtenues à partir du courant de produits et qui contiennent des hydrocarbures qui présentent au plus un nombre de carbone de 5 sont recyclées dans le second four de clivage (2).

6. Procédé selon l'une quelconque des revendications 1 à 5, **caractérisé en ce qu'**une ou plusieurs fractions (U) qui sont obtenues à partir du courant de produits et qui contiennent des hydrocarbures qui présentent au moins un nombre de carbone de 6 sont recyclées dans le premier four de clivage (1).

7. Procédé selon l'une quelconque des revendications 1 à 6, **caractérisé en ce qu'**en plus de la seconde fraction d'alimentation fraîche (B2), une alimentation fraîche supplémentaire (BL) est introduite dans le second four de clivage (2), qui est principalement constituée d'hydrocarbures qui présentent au plus un nombre de carbone de 5.

8. Procédé selon l'une quelconque des revendications 1 à 7, dans lequel la température de sortie du four de clivage pour la réaction dans le second four de clivage (2) est comprise entre 680 °C et 820 °C, de préférence entre 700 °C et 800 °C, et de manière davantage préférée entre 710 °C et 780 °C, et de manière particulièrement préférée entre 720 °C et 760 °C, et la température de sortie du four de clivage pour la réaction dans le premier four de clivage (1) est comprise entre 800 °C et 1 000 °C, de préférence entre 820 °C et 950 °C, et de manière particulièrement préférée entre 840 °C et 900 °C, la température de sortie du four de clivage du premier four de clivage (1) étant supérieure à celle du second four de clivage (2).

9. Procédé selon la revendication 8, dans lequel la température de sortie du four de clivage pour la réaction dans le premier four de clivage (1) est supérieure d'au moins 10 °C, de préférence d'au moins 15 °C, de manière particulièrement préférée d'au moins 20 °C, à la température de sortie du four de clivage pour la réaction dans le second four de clivage (2).

10. Procédé selon l'une quelconque des revendications 1 à 9, dans lequel 0,3 à 1,5 kg de vapeur d'eau par kg d'alimentation hydrocarbonée est utilisée dans le premier four de clivage (1) et 0,15 à 0,8 kg de vapeur d'eau par kg d'alimentation hydrocarbonée dans le second four de clivage (2).

11. Procédé selon l'une quelconque des revendications 1 à 10, dans lequel au moins une fraction (V), qui comprend principalement des hydrocarbures d'un nombre de carbone de 2 ou 3, est obtenue à partir du courant de produits, et au moins partiellement transformée dans un four de clivage (3) pour alimentation gazeuse.

12. Procédé selon l'une quelconque des revendications 1 à 11, **caractérisé en ce que** des condensats de gaz naturel et/ou des fractions de pétrole, notamment du naphta, et/ou des hydrocarbures synthétiques et/ou biogènes et/ou des mélangés dérivés de ceux-ci sont utilisés en tant qu'alimentation fraîche (B).
